# EUROPEAN PATENT APPLICATION

(11) **EP 2 886 552 A1**
(43) Date of publication of application: **24.06.2015**
(21) Application number: 13197794.4
(22) Date of filing: 17.12.2013
(51) Int. Cl.: C07K 14/47

(54) **Methods for purging isolated organs and cells from latent human cytomegalovirus**

(71) Applicant: Ecole Polytechnique Federale de Lausanne (EPFL), 1015 Lausanne (CH)
(72) Inventor: Trono, Didier, 1134 Vufflens-le-Chateau (CH); Rauwel, Benjamin, 74200 Thonon-les-Bains (FR)
(74) Representative: Cabinet Plasseraud

(57) **Abstract**

The present invention relates to a method for purging *ex vivo* isolated organs or isolated cells from latent human cytomegalovirus (HCMV). The invention further relates to a compound for use in a method for treating HCMV infection.

## Description

### FIELD OF THE INVENTION

The present invention relates to a method for purging *ex vivo* isolated organs or isolated cells from latent human cytomegalovirus (HCMV). The invention further relates to a compound for use in a method for treating HCMV infection.

### BACKGROUND OF THE INVENTION

Human cytomegalovirus (HCMV) is a β-herpesvirus (Human herpesvirus 5, genus Cytomegalovirus, subfamily Betaherpesvirinae, family Herpesviridae). HCMV is a highly prevalent pathogen that induces life-long infections by establishing latency in hematopoietic stem cells (HSC) and monocytes. The capacity to establish cellular sites of viral latency while avoiding recognition and elimination by the host immune system is critical to the ability of HCMV to maintain life-long infection.

Latent cytomegalovirus is frequently transmitted by organ transplantation, and its reactivation under conditions of immunosuppressive prophylaxis against graft rejection by host-versus-graft disease bears a risk of graft failure due to viral pathogenesis. Indeed, HCMV-negative recipients of latently HCMV infected donor grafts are at highest risk for developing CMV disease. Clinical studies have directly associated HCMV infection with the acceleration of solid organ allograft chronic rejection due, inter alia, to increased transplant vascular sclerosis. Thus, latent CMV constitutes a high risk factor for graft failure long after transplantation.

It has been shown that when a graft from a CMV latently infected donor is transplanted into an uninfected recipient, the immediate environment is one of inflammation due to ischemia/reperfusion and the obligatory immune suppression therapy given at the time of transplantation to prevent acute graft rejection. This environment promotes CMV reactivation, which is considered to be the major source of viral infection. Thus, the presence of HCMV, whether asymptomatic or manifesting over infection, has been found to be a negative impact on graft survival. Bouts of reactivation are normally controlled by the immune system, but can be life-threatening in AIDS patients and immunocompromised patients receiving blood transfusions or organ or bone marrow grafts.

Therefore, there is an unfulfilled need for a method for eradicating HCMV from a graft and an efficient therapeutic strategy for treating patient suffering from CMV infection.

### SUMMARY OF THE INVENTION

The inventors have shown an essential role of KAP1 for the establishment and the maintenance of human cytomegalovirus (HCMV). More precisely, they have shown that the virus exits latency when KAP1 become phosphorylated on serine 824. The inventors further identified mTOR as responsible for this KAP1 phosphorylation switch. They thus found out that the HCMV latency can be externally modulated by agent targeting this specific enzyme or ATM, which is another KAP1 kinase. Furthermore, the inventors developed a promising strategy for eradicating infection by combining mTOR or ATM activation with immune or drug-based approach aimed at killing HCMV infected cells.

Consequently, in a first aspect, the invention relates to the use of a composition for purging *ex vivo* isolated organs or isolated cells from latent human cytomegalovirus (HCMV), wherein said composition comprises:
- an agent selected in the group consisting of a KAP1 protein with a phosphorylated serine on position 824, a kinase which phosphorylates KAP1 on serine 824, an activator of said kinase, and a phosphatase inhibitor targeting KAP1 on serine position 824; and
- an anti-CMV agent.

In addition, by figuring out the biological mechanism responsible for latency in HCMV, the inventors developed a promising therapeutic strategy for treating HCMV infection. Consequently, in a second aspect, the invention relates to a compound for use in a method for treating HCMV infection by maintaining or forcing latency, wherein said compound prevents KAP1 phosphorylation on serine 824.

### DETAILED DESCRIPTION OF THE INVENTION

The inventors found out that KRAB/KAP-mediated regulation plays a critical role in the biology of human cytomegalovirus (CMV), a highly prevalent viral pathogen. After an often lowly symptomatic primary infection, human CMV establishes a latent reservoir notably in hematopoietic stem cells (HSC), leading to life-long persistence.

The mechanisms of CMV latency are poorly understood, but the viral genome is known to persist as a non-replicating episome adorned with repressive histone marks, notably H3K9me3. The inventors found out that KRAB/KAP-mediated repression is functional within the context of episomal DNA. Furthermore, the KRAB/KAP pathway has been found to influence some aspects of the replication of two other members of the herpes virus family, Epstein-Barr virus (EBV) and Kaposi's sarcoma-associated herpes virus (KSHV). Based on these premises and capitalizing on their parallel efforts to study the role of KRAB/KAP in hematopoiesis, the inventors investigated whether this system influences hCMV replication.

Starting from this point, the inventors met the burden to identify KAP1 as a key determinant of CMV latency, based on the following evidence:
i) KAP1 knockdown prevents both the establishment and the maintenance of HCMV latency in human CD34⁺ HSC;
ii) in HSC infected with the clinical strain TB40-E of CMV (capable of instating latency), KAP1 binds to some 28 sites of the viral genome, where it recruits the histone methyltransferase SETDB 1, triggering H3K9 trimethylation;
iii) in MRC5 fibroblasts (where CMV infection is lytic) or in HSC infected with the laboratory strain AD169 of CMV (unable to establish latency), KAP1 is also tethered to the viral genome, but without SETDB1 and H3K9 trimethylation;
iv) explaining this difference, the form of KAP1 recruited to the viral genome in these latter settings is phorphorylated on serine 824, a modification previously reported to prevent SETDB1 binding;
v) correspondingly, latency can be prevented in HSC by overexpression of the phosphomimetic mutant with a glutamic acid on position 824;
vi) the mediator of KAP1 phosphorylation in CMV-infected cells is not ATM, a kinase known to target the corepressor within the setting of DNA damage, but mTOR, the activation of which in CMV-infected cells was previously documented and demonstrated to be necessary for lytic replication;
vii) hCMV reactivation is curtailed in AD169-infected CD34⁺ HSC treated with the mTOR inhibitor Torin1;
vii) hCMV is forced out of latency in TB40-E-infected CD34⁺ HSC treated with the mTOR activator MHY1485 or the ATM activator chloroquine.

Thus, accordingly, in a first aspect, the invention relates to the use of a composition for purging *ex vivo* isolated organs or isolated cells from latent human cytomegalovirus (HCMV), wherein said composition comprises:
- an agent selected in the group consisting of a KAP1 protein with a phosphorylated serine on position 824, a kinase which phosphorylates KAP1 on serine 824, an activator of said kinase, and a phosphatase inhibitor targeting KAP1 on serine position 824; and
- an anti-CMV agent.

The inventors have indeed developed a promising strategy for forcing HCMV out of transcriptional dormancy in latently infected cells.

As used herein, **"KAP1",** also known as **"TRIM28"** or **"TIF1β",** serves as universal corepressor to the large family of KRAB-containing zinc finger proteins, and as such partakes in the early embryonic silencing of endogenous retroelements as well as in the regulation of many physiological pathways, including in the hematopoietic system. The inventors previously also demonstrated that KRAB/KAP-mediated repression is functional within the context of episomal DNA. Last, the KRAB/KAP pathway has been found to influence some aspect of the replication of two other members of the herpes virus family, Epstein-Barr virus (EBV) and Kaposi's sarcoma-associated herpes virus. The sequence of KAP1 is available under the NCBI Reference Sequence: NP_005753.1.

As used herein, **"latency, "dormancy"** or **"persistence"** can be used interchangeably and are defined operationally as the persistence of the viral genome in the absence of production of infectious virions, but with the ability of the viral genome to reactivate under specific stimuli.

As used herein, unless it is qualified otherwise, the expression **"agent"** alone or "agent of the invention" or "agent of the composition of the invention" refers to:
- a KAP1 protein with a phosphorylated serine on position 824,
- a kinase which phosphorylates KAP1 on serine 824,
- an activator of kinase which phosphorylates KAP1 on serine 824, or
- a phosphatase inhibitor targeting KAP1 on serine 824.

The composition of the invention is used *ex vivo.* The use thus takes place outside an organism. The use allows purging latently infected cells by HCMV outside the animal or human body. The ex vivo use according to the invention on HCMV-positive transplant, for instance bone marrow or HCS purified therefrom, constitutes an highly promising strategy for purging latenly infected cells prior to engraftment.

In one embodiment, the agent of the composition is a kinase selected in the group consisting of mTOR and ATM.

As used herein, **"ATM"** or "ataxia telangiectasia mutated" refers to a serine-protein kinase. It refers to a kinase which phosphorylates KAP1 at position 824. Said phosphorylation results in decreased KAP1 sumoylation and SETDB1 recruitment, with secondary loss of KAP1 repressor activity. Correspondingly, a S824D mutation of KAP1, which mimics constitutive phosphorylation of the protein, similarly leads to decreased KAP1 sumoylation and repressive potential. The sequence of ATM is available under the accession number UniProtKB/Swiss-Prot: Q13315.3.

As used herein, **"mTOR"** refers to a Serine/threonine-protein kinase. It refers to the mammalian target of rapamycin. Activation of this kinase occurs during HCMV replication. The sequence of mTOR is available under the accession number UniProtKB/Swiss-Prot: P42345.1.

Either ATM or mTOR can induce S824 phosphorylation.

As used herein, the expressions **"824 phosphorylation"** or **"phosphorylation** of **KAP1 on serine 824"** refer to the presence or the addition of a phosphate on the serine at position 824 of the amino acid sequence of KAP1, available under the NCBI Reference Sequence: NP_005753.1.

As used herein, the expression **"KAP1 phosphomimetic mutant"** refers a KAP1 protein bearing a mutation at position 824 that mimics a phosphorylated serine (e.g. aspartic or glutamic acid).

Activators of kinase which phosphorylates serine at position 824 of KAP1 can also be used for purging *ex vivo* isolated organs or isolated cells from latent human cytomegalovirus. Therefore, in another embodiment, the agent of the composition is an activator of the kinase selected from activators of mTOR and activators of ATM.

The inventors have shown the efficiency of said activators, by using :
- chloroquine, or
- MHY-1485.

Therefore, said activator of the kinase is preferably selected from MHY-1485 and chloroquine.

Inhibitors of phosphatase targeting KAP1 on serine 824 can also be used for purging *ex vivo* isolated organs or isolated cells from latent human cytomegalovirus.

Typically, said inhibitors are inhibitors of a phosphatase selected from PP1B and PP4C. Therefore, in another embodiment, the agent of the composition is an inhibitor of phosphatase targeting KAP1 on serine 824, such as an inhibitor of PP1B or an inhibitor of PP4C. Preferably, said inhibitor of phosphatases targeting KAP1 on serine 824 is okadaic acid.

As used herein, **"PP1B"** refers to the serine/threonine-protein phosphatase PP1-beta catalytic subunit isoform 1, known to be involved in the regulation of a variety of cellular processes, such as cell division, glycogen metabolism, muscle contractility, protein synthesis, and HIV-1 viral transcription. The sequence of PP1B is available under NCBI Reference Sequence: NP_996759.1.

As used herein **"PP4C"** refers to the serine/threonine-protein phosphatase 4 catalytic subunit. The sequence of PP4C is available under NCBI Reference Sequence: NP_002711.1.

As used herein, the expressions **"anti-CMV agent"** or **"anti-CMV agent of the composition"** refer to an agent which aims to kill HCMV-infected cells. For achieving said purpose, the anti-CMV agent of the composition relies on immune-based approach or drug-based approach. Preferably, the anti-CMV agent of the composition is selected from monoclonal antibodies, antiviral drugs and cytotoxic agents. Preferably, said anti-CMV agent is a cytotoxic agent. More preferably, said anti-CMV agent is vincristine. As used herein, the terms **"vincristine", "leurocristine", or "VCR"** refers to a vinca alkaloid from the *Catharanthus roseus.* It is a mitotic inhibitor, and is used in cancer chemotherapy. Vincristine is created by the coupling of indole alkaloids vindoline and catharanthine in the vinca plant.

In one embodiment, the composition of the invention is used to purge *ex vivo* an isolated organ from HCMV. More preferably, said organ is a graft organ. As used herein, the expressions **"graft organ"** and **"transplant"** can be used interchangeably and refer to an organ which is intended to be engrafted. Said organ can be any organ which can be grafted. Typically, said transplant is a solid graft. A non limiting list of grafts include, but are not limited to: heart, kidney, lung, liver, pancreas, pancreatic islets, brain tissue, stomach, large intestine, small intestine, cornea, skin, trachea, bone, bone marrow, muscle, or bladder. Preferably, the grafts that can be purged from HCMV with the composition of the invention are bone marrow, kidney, liver, pancreas, heart, and lung.

In another embodiment, the composition of the invention is used to purge *ex vivo* isolated cells from HCMV. Typically, said cells are blood or bone marrow cells. Preferably, said cells are hematopoietic stem cells.

The agent and the anti-CMV agent of the composition of the invention can be put in contact with the isolated organ or isolated cell to be purged from latent HCMV in a simultaneous, separate or sequential manner.

The invention also relates to a method for purging isolated organs or isolated cells from latent human cytomegalovirus (HCMV), said method comprises the step of putting in contact said isolated organs or isolated cells with a composition comprising:
- an agent selected in the group consisting of a KAP1 protein with a phosphorylated serine on position 824, a kinase which phosphorylates KAP1 on serine 824, an activator of said kinase, and a phosphatase inhibitor targeting KAP1 on serine position 824; and
- an anti-CMV agent.

The method of the invention can also comprise a prior step of detecting whether said isolated organs or isolated cells or donor thereof is infected with HCMV. HCMV infection may be detected with any standard methods well known in the art. Typically, said detection may be performed by antibody detection techniques such as complement-fixation techniques, enzyme-linked immunosorbent assays (ELISA), latex agglutination, or radioimmunoassays.

All the previously mentioned technical data are applicable here.

The inventors also put in light the molecular mechanisms of HCMV latency, and thus developed a therapeutic strategy for preventing HCMV to replicate. Consequently, in a second aspect, the invention relates to a compound for use in a method for treating HCMV infection, wherein said compound prevents KAP1 phosphorylation on serine 824.

As used herein, the expression **"method for treating HCMV infection"** refers to a method for preventing HCMV replication. The compound of the invention is thus highly useful for:
- forcing HCMV to enter latency, and/or
- maintaining HCMV into latency.

Therefore, by preventing replication of HCMV, the clinical situation of a patient is improved.

In a further embodiment, the invention relates to a compound and an anti-CMV agent for use in a method for treating HCMV infection, wherein said compound prevents KAP1 phosphorylation on serine 824. Typically, said compound and said anti-CMV agent can be used as a combined preparation for simultaneous, separate or sequential use in a method for treating HCMV infection.

Typically, said compound is selected in the group consisting of:
- inhibitors of a kinase which phosphorylates KAP1 on serine 824, and
- inhibitors of activators of kinases which phosphorylate KAP1 on serine 824,
- phosphatases targeting KAP1 on position 824, and
- activators of phosphatases targeting KAP1 on position 824.

Preferably, said compound is an inhibitor of a kinase which phosphorylates KAP1 on serine 824, said kinase being selected from mTOR and ATM.

More preferably, said compound is an inhibitor of mTOR, typically Torin1.

In another embodiment, said compound is a phosphatase targeting KAP1 on position 824 selected from PP1B and PP4C.

In yet another embodiment, said compound is an activator of phosphatases targeting KAP1 on position 824, preferably ceramide.

In a specific embodiment, the invention relates to a compound for use in a method for treating HCMV infection in an immune-depressed subject, wherein said compound prevents KAP1 phosphorylation on serine 824.

Typically, said immune depressed subject suffers from HIV infection or AIDS or is a subject who has gone through organ transplantation. Indeed, reactivation of a latent HCMV in said patients, who are immunocompromised and who are highly sensitive, can be life threatening.

The compound of the invention as above described may be combined with pharmaceutically acceptable excipients, and optionally sustained-release matrices, such as biodegradable polymers, to form therapeutic compositions.

**"Pharmaceutically"** or **"pharmaceutically acceptable"** refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, intraocular, intravenous, intramuscular or subcutaneous administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

To prepare pharmaceutical compositions, an effective amount of the compound of the invention may be dissolved or dispersed in a pharmaceutically acceptable carrier or aqueous medium.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, mixtures thereof and in oils. Under ordinary conditions of storage and use, these preparations contain a preservative to prevent the growth of microorganisms.

The compound of the invention can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution may be suitably buffered and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

In addition to the compounds formulated for parenteral administration, such as intravenous or intramuscular injection, other pharmaceutically acceptable forms include, e.g. tablets or other solids for oral administration; time release capsules; and any other form currently used.

The invention will be further illustrated by the following figures and examples. However, these examples and figures should not be interpreted in any way as limiting the scope of the present invention.

### FIGURE LEGENDS

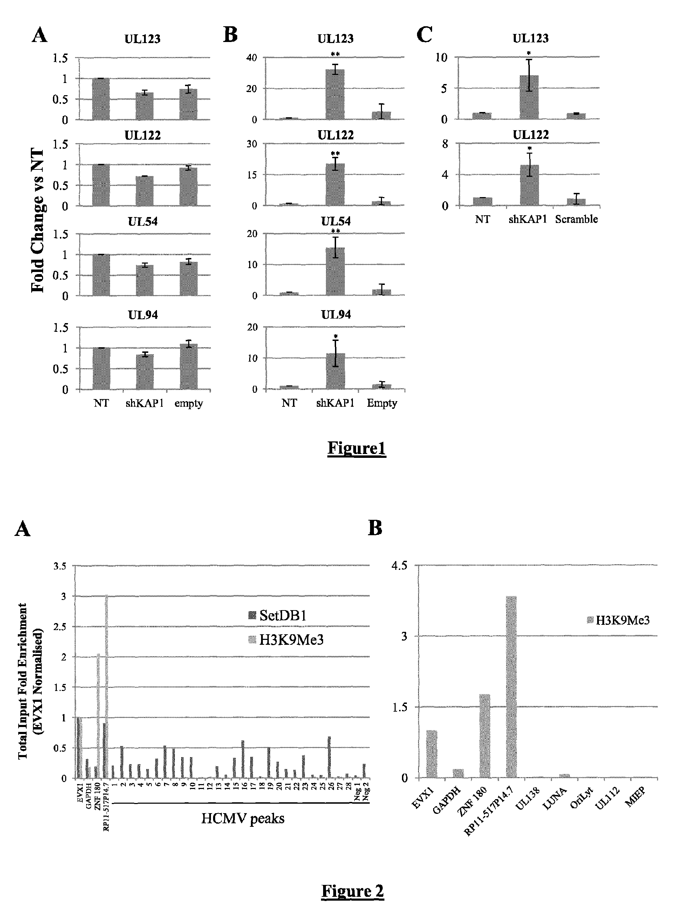

**Figure 1****:** KAP1 is required for HCMV latency. RT-qPCR analysis of indicated HCMV genes expression in MRC-5 (A) or CD34+ (BC) cells infected with the TB40-E strain 3 days after (AB) or 7 days prior to (C) being transduced or not (NT) with lentivectors expressing or not (empty) a small hairpin RNA against Kap1 (shKAP1). Results are presented as fold change expression versus NT after GAPDH and β-2M normalization (n=4, *p<0.05, **p<0.01).

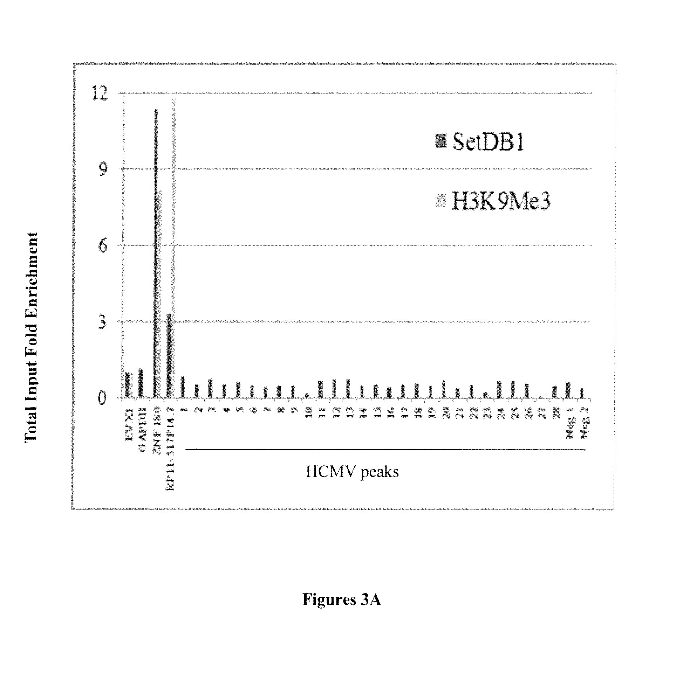

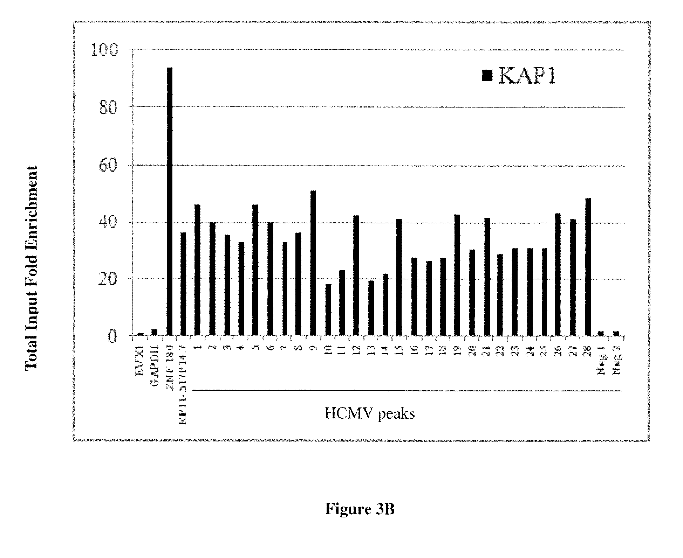

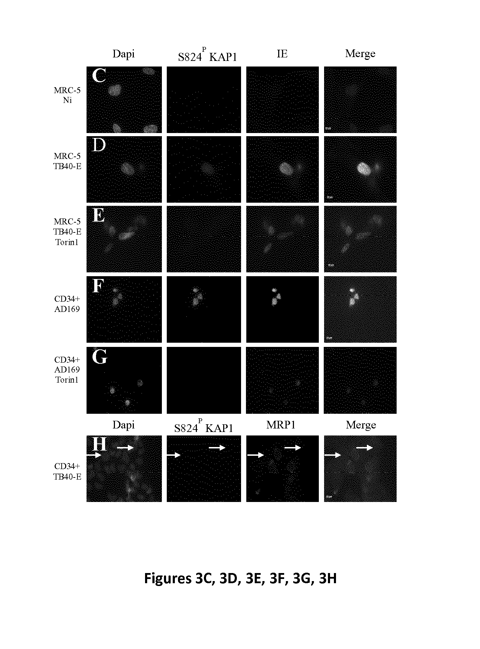

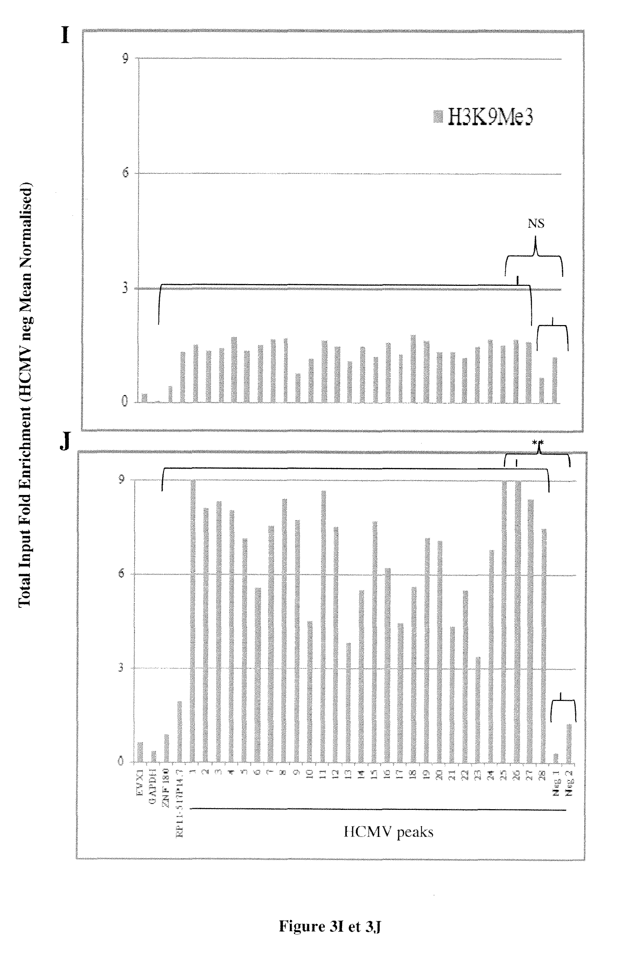

**Figure 2****:** Loss of SETDB1 recruitment and H3K9 trimethylation in HCMV-infected HSC depleted for KAP1. ChIP-PCR analysis of TB40-E-infected KAP1-depleted CD34+ with SETDB1(A) and H3K9Me3 (A, B) specific antibodies was performed as described in Fig. 3 legend.
**Figure 3****:** An mTOR-mediated KAP1 phosphorylation switch governs HCMV progression from latency to lytic replication. ChIP-PCR for indicated HCMV genomic regions were performed with anti-SetDB1 and anti-H3K9Me3 (A), anti-KAP1 (B) or anti-S824 phosphoKAP1 antibodies (I, J) on material extracted from MRC-5 (A, B, J) or CD34+ (I) TB40-E-infected cells, using same controls as in Fig. 2. Results are presented as total input fold enrichment after EVX-1 normalization. MRC-5 (C, D, E) and CD34+ (F, G, H) cells, infected (TB40-E, AD169) or not (Ni) with HCMV, and treated or not with mTOR inhibitor (Torin1) were examined by immunofluorescence with anti-IE and anti-MRP1 antibodies (Alex-488, green), and anti-S824 phosphoKAP1 antibodies (Alexa 568, red). DNA was stained with Dapi (blue). White arrows represent latently infected cells (MRP-1 negative cells). All pictures are representative of slide overview from 3 independent experiments.

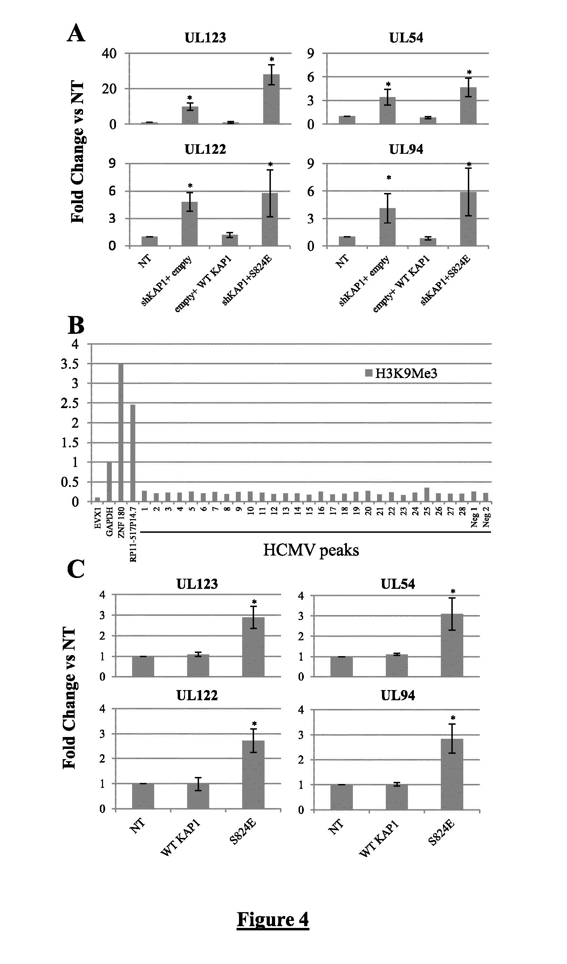

**Figure 4****:** HCMV can be forced out of latency by KAP1 genetic manipulation.
   (A) RT-qPCR analysis of indicated HCMV transcripts in CD34+ cells infected with the TB40-E strain, three days after transduction or not (NT) with lentiviral vectors expressing or not (empty) an shRNA (shKAP1) against Kap1, together with a vector expressing an shRNA-resistant allele of the S824 KAP1 phosphomimetic mutant (S824E) or a wild type KAP1 (WT KAP1) lentiviral vector. Results are presented as fold change expression versus NT after GAPDH and β-2M normalization (n=3, *p<0.05). The S824E KAP1 mutant does not rescue latency after endogenous KAP1 knockdown.
   (B) Anti-H3K9Me3 ChIP analysis of KAP1-depleted CD34+ cells infected with TB40-E and complemented with S824E KAP1. See Fig. 1 for details. The repressive mark is not restored by the KAP1 phosphomimetic mutant.
   (C) Same as in (A), without KAP1 knockdown but using lentiviral vectors to overexpress with wild-type or S824 phosphomimetic versions of KAP1. The S824E KAP1 mutant exerts a dominant negative effect on endogenous KAP1-induced HCMV latency.

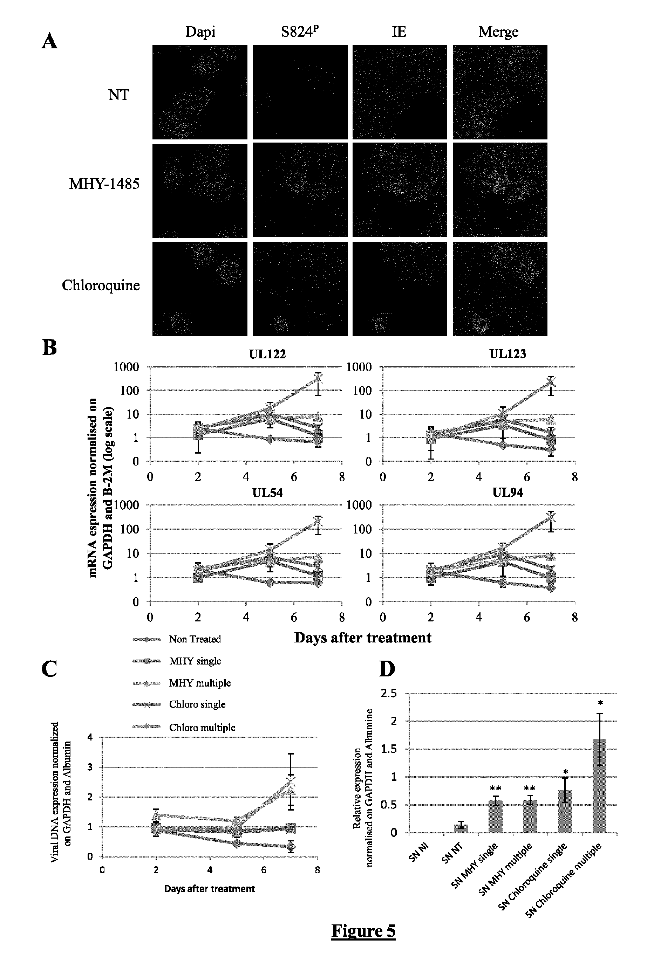

**Figure** 5: Forcing HCMV out of latency by KAP1 pharmacological manipulation. After 5 days of infection with TB40-E, CD34⁺ cells were treated or not (mock) with mTOR (MHY-1485) or ATM (Chloroquine) at single (single) or multiple doses (multiple) as indicated. (A) After 5 days of pharmacological treatment, immuno-fluorescent staining was performed for S824 phosphoKAP1 (S824^{P}) and HCMV IE1-2 (IE). (B) Indicated HCMV transcripts were quantified by RT-qPCR, using GAPDH and β-2 Microglobulin for normalization. (C) HCMV DNA associated with the TB40-E-infected HSC was quantified by qPCR, normalizing with the GAPDH and albumin genes. Data are presented as average of 3 different experiments performed with cells from independent donors. (D) Supernatant from the TB40-E-infected CD34⁺ cells, harvested after 7 days of treatment, was used to infect MRC-5 fibroblasts, which were assessed for their viral DNA content 3 days later by qPCR. Histogram represents an average of 3 different experiments performed with HSC from 3 independent donors (n=3, *p<0.05, **p<0.01).

### EXAMPLE

Human cytomegalovirus (HCMV), a member of the β-herpes virus family, is a highly prevalent pathogen that induces life-long infections through the establishment of latency in hematopoietic stem cells (HSC) and monocytes (Sinclair, 2008). Bouts of reactivation are normally asymptomatic because rapidly controlled by the immune system, but can be fatal in immuno-compromised individuals such as AIDS patients and transplant recipients (Sissons and Carmichael, 2002). Furthermore, primary HCMV infection during pregnancy is a leading cause of congenital malformations of the central nervous system (Britt, 2008).

The approximately 250kb genome of HCMV encodes several hundred proteins, 14 miRNAs and a few long-noncoding RNAs (Stern-Ginossar et al., 2012). Infection of permissive targets such as epithelial cells or fibroblasts leads to a lytic cycle, with a highly orderly transcriptional cascade that first expresses the viral immediate early (IE) genes, the products of which set the cellular stage for the virus and activate the viral early (E) genes; these yield the effectors of viral genome replication, before proteins encoded by the viral late (L) genes finally trigger the formation of new particles. In contrast, when HCMV infects a HSC, an early myeloid precursor or a monocyte, the expression of lytic genes is suppressed and only a few latency-associated transcripts are detected (Goodrum et al., 2012). The viral genome is then maintained as a stable episome, without replicating or inducing the production of new virions. The molecular mechanisms of HCMV persistence are poorly understood, but known to be associated with epigenetic modifications of the viral genome (Avdic et al., 2011; Mason et al., 2012; Petrucelli et al., 2012; Umashankar et al., 2011). In latently infected CD34+ HSC or circulating monocytes isolated from seropositive individuals, chromatin at the major immediate early promoter (MIEP) bears histone 3 trimethylated on lysine 9 (H3K9me3), a repressive mark, and heterochromatin protein 1 (HP1) (Reeves and Sinclair, 2013 ; Sinclair, 2010). Upon differentiation of latently infected precursors into dendritic cells, H3K9me3 is replaced by high levels of histone acetylation, HP1 is lost, and a lytic cycle is triggered (Hahn et al., 1998 ; Mendelson et al., 1996; Reeves et al., 2005; Taylor-Wiedeman et al., 1994). KAP1 (KRAB-associated protein 1), also known as TRIM28 (tripartite motif protein 28) or TIF1β(transcription intermediary factor 1 beta) is a transcriptional co-repressor that is essential for the early embryonic silencing of endogenous retroelements (Rowe et al., 2010; Wolf and Goff, 2007) and involved in regulating multiple aspects of mammalian homeostasis, including in the hematopoietic system (Barde et al., 2013; Bojkowska et al., 2012 ; Chikuma et al., 2012; Jakobsson et al., 2008 ; Santoni de Sio et al., 2012a; Santoni de Sio et al., 2012b). KAP1 binds to the Kruppel-associated box (KRAB) domain present at the N-terminus of KRAB-containing zinc finger proteins (KRAB-ZFPs) (Friedman et al., 1996). These constitute the single largest family of transcriptional repressors encoded by the genomes of higher organisms, with close to four-hundred members in either human or mouse, and are endowed with sequence-specific DNA binding ability via a C-terminal array of zinc fingers (Urrutia, 2003). KAP1 harbors from its N- to its C-terminus a RING finger, B-boxes, a coiled coil region, a HP1-binding motif, a PHD finger and a bromodomain (Iyengar and Farnham, 2011). The first three of these motifs define the so-called RBCC or TRIM (tripartite motif) region, which is both necessary and sufficient for homo-oligomerization and direct binding to KRAB. The C-terminal effector end of the protein recognizes the backbone of histone tails, and interacts with two histone-modifying enzymes: Mi2α, an isoform of the Mi2 protein found in the NuRD (nucleosome remodeling and histone deacetylation) complex (Schultz et al., 2001), and SETDB1 (SET domain, bifurcated 1), a H3K9me3-specific histone methyltransferase (Schultz et al., 2002). The H3K9me3 mark in turn creates high affinity genomic binding sites for HP1, bringing more KAP1 complex, which likely explains that KAP1-induced heterochromatin formation can spread several tens of kilobases away from an initial KAP1 docking site (Groner et al., 2010). SETDB1 recruitment is stimulated by sumoylation of the KAP1 bromodomain, the last step of which is mediated intramolecularly by an E3 ligase activity contained in the PHD domain (Ivanov et al., 2007). Within the context of DNA damage, the ATM (ataxia telangiectasia mutated) kinase phosphorylates KAP1 at position 824, resulting in decreased KAP1 sumoylation and SETDB1 recruitment, with secondary loss of KAP1 repressor activity (Noon et al., 2010 ; White et al., 2012; White et al., 2006 ). S824D/E mutations of KAP1, which mimic constitutive phosphorylation of the protein, similarly lead to decreased KAP1 sumoylation and repressive potential. Histone 3 lysine 9 trimethylation deposition and HP1 recruitment are thus signatures of KAP1 action. Furthermore, the inventors previously demonstrated that KRAB/KAP-mediated repression is functional within the context of episomal DNA (Barde et al., 2009). Finally, the KRAB/KAP pathway has been found to influence the replication of two other members of the herpes virus family, Epstein-Barr virus (EBV) (Liao et al., 2005) and Kaposi's sarcoma-associated herpes virus (KSHV) (Cai et al., 2013; Chang et al., 2009)}.

Based on these premises, the inventors investigated a possible role for KAP1 in the control of HCMV latency.

### Material and methods

**Cell culture and treatments.** CD34+ cells from human cord blood were obtained from the Lausanne University Hospital (Centre Hospitalier Universitaire Vaudois, CHUV) delivery room with proper informed consent, layered on Ficoll gradient, purified with the CD34+ cell separation kit (Miltenyi Biotec) and freshly used for experiments without freezing to avoid differentiation. CD34+ cells were stimulated for one day in X-Vivo 15 medium (Lonza) supplied with cytokines (100 ng/ml of Flt-3 ligand, 100 ng/ml of SCF, 20 ng/ml of TPO, and 20 ng/ml of IL-6; Peprotech) and 5% penicillin/streptomycin, maintained at a density of 5.10₅ cells/mL and transduced the day after stimulation with lentiviral vector at a MOI of 100 as previously described (Barde et al., 2013), washed 24 hrs later and cultured in X-Vivo 15 medium, 50 ng/ml of Flt-3 ligand, 25 ng/ml of SCF and 20 ng/ml of TPO. MRC5 fibroblasts (ATCC CCL-171) were propagated in DMEM medium (InVitrogen) containing 10% fetal calf serum and 5% penicillin/streptomycin. MRC-5 fibroblasts were transduced with a lentivector at a MOI of 10 3 days before HCMV infection. The mTOR (Torin1 and rapamycin, Selleckchem) and ATM (KU55933, LuBioscience) inhibitors were used at 0.1 and 10 µM, respectively, added 30 min before infection. The mTOR (MHY-1485, Glixx Lab) and ATM (chloroquine, Sigma-Aldrich) activators were used at 10 and 20µM respectively.

**Lentiviral vectors.** pLKO vectors were purchased from Sigma-Aldrich and the puromycin was replaced by eGFP (pLKO-shKAP1, pLKO-empty, pLKO-scramble). QuickChange II XL Site directed Mutagenesis Kit (Agilent Technologies) was used to mutate KAP1 Serine 824 into Glutamic Acid in a pFUT plasmid containing a human *Kap1* allele modified by nucleotide substitutions to resist to our shRNA. Production and titration of lentiviral vectors was performed as previously described (Barde et al., 2011).

Viruses. The AD169 (ATCC) and TB40-E (a gift from G. Herbein, Besançon, France) HCMV strains were used in this study. Virus stocks were generated in MRC5 or HUVEC cells, collecting particles when cytopathic effects were > 90%. Supernatants were clarified of cell debris by centrifugation at 1,500g for 10 min, ultracentrifuged at 100,000g for 30 min at 4° C and stored at -80°C until use. Virus titers were determined by plaque assay on MRC5 cells using standard methods. MRC-5 were infected at a MOI of 1, while CD34+ infection were performed at a MOI of 5, 3 days after or 7 days before transduction with lentivectors.

**Chromatin immunoprecipitation (ChIP).** Chromatin from 10₇ CD34+ was prepared and immunoprecipitated as in (Barde et al., 2013), with KAP1- (Abcam), H3K9me3-(Diagenode), SETDB1- (Abcam) or S₈₂₄ phosphoKAPl- (Abcam) specific antibodies. TB40-E infected CD34+ KAP1-ChIPed DNA was sent to sequencing. The 50bp reads for KAP1 IP and Total Input were mapped to the HCMV genome (TB40-BAC4 GenBank accession EF999921.1) using the bowtie short read aligner version 0.12.7 (Langmead et al., 2009) and allowing up to 3 mismatches over the whole length of the read. The signal of the total input was then subtracted from the KAP1 IP signal using a custom made PERL program and regions with similar signal were merged together. The resulting regions were ranked according to their signal. The signal follows a normal distribution (tested with Shapiro-Wilk normality test) thus the inventors considered as enriched regions the extreme right part of the distribution. SYBR green qPCR was performed to quantify enrichment at specific loci. Cellular promoters regions were used as negative (EVX-1; GAPDH) or positive (ZNF-180;RP11-517P14.7) controls for PCR enrichment. 28 highly enriched peaks were found for the HCMV TB40-E strain, and 2 negative regions were designed for qPCR controls.

**ImmunoFluorescence.** MRC-5 were cultured directly on coverslips. Around 2x10₅ CD34+ were attached to slides by cytospinning with Shandon EZ Single Cytofunnel (Histocom). Cells were then fixed and permeabilised with Methanol during 5 minutes at - 20°C, and saturated with PBS 5% FCS during 2 hours at room temperature (RT). Primary staining with IE-, anti-S₈₂₄ phopshoKAP1-, KAP1- (all from Abcam) anti-KAP1 S₄₇₃ phospho KAP1 (BioLegend) specific antibodies was performed in PBS 5% FCS at 4°C overnight or 3 hours at RT, before addition of secondary antibodies (Alexa-488 or -565) in PBS 5% FCS during 1 hour at RT. Dapi bath was performed during 10 min at RT, and slides were mounted with Fluoromount-G (Southern Biotech).

**RT-PCR.** Cells were sorted for GFP (control of transduction) and CD34 expression. RNA was extracted by Trizol and reverse transcribed with Superscript II (both from Invitrogen) according to the manufacturer's instructions. All qPCR were performed with SYBR green mix (Roche). Primers used in this work were designed by Primer Express software (Applied Biosystems) and their sequence can be provided upon request.

**Western Blot Analyses.** Cells lysates were subjected to SDS/PAGE on 4-12% polyacrylamide gels (InVitroGen). iBlots on Nitrocellulose membrane (InVitrogen) were treated with KAP1-(Abcam) and βactin-(Calbiochem) specific antibodies, followed by polyclonal rabbit HRP-conjugated antibody, and protein bands were detected by using an ECL-plus kit (Thermo-Scientific).

### RESULTS

### KAP1 is necessary for both establishment and maintenance of HCMV latency in HSC

To examine the possible impact of KAP1 on HCMV replication, the inventors first depleted the corepressor by lentivector-mediated RNA interference in MRC-5 fibroblasts, a cell type fully permissive for HCMV replication. Cells transduced with either a *Kap1* knockdown (shKap1) or a control GFP-expressing vector were infected with HCMV, and 3 days later the expression of viral genes indicative of a lytic cycle (the IE genes UL123, UL122, the E gene UL54 and the L gene UL94) was quantified by RT-qPCR (Fig. 1A). No significant differences were observed, indicating that KAP1 is not essential for productive HCMV replication. The inventors then turned to a cellular model of HCMV latency. Human CD34+ cells were purified from cord blood and transduced with either the control or the *Kap1* knockdown vector (Fig. S1A, S1B), and infected 3 days later with the TB40-E HCMV clinical strain, known to induce latency in these targets (Goodrum et al., 2007). At 7 days post-infection, viral mRNA levels in CD34+GFP+ sorted cells were analyzed by RT-qPCR. Immediate early, early and late genes were markedly more highly expressed (between 10 and 35 fold) in *Kap1* knockdown than in control cells (Fig.1B). Furthermore, upon complementation of KAP1-depleted cells with an shRNA-resistant *Kap1* allele, latency was restored (Fig. S1C-G), demonstrating that, in knockdown cells, HCMV entry into lytic phase was truly due to decreased KAP1 levels. These results thus indicated that KAP1 is necessary for the establishment of HCMV latency in HSC. The inventors then inverted the sequence of our manipulations to induce *Kap1* knockdown in CD34+ cells that had been infected 7 days earlier with the TB40-E virus (Fig. S1H). Three days later, the inventors measured the expression of the UL122 and UL123 mRNAs, which are found only during a lytic cycle (Reeves and Sinclair, 2013) (Fig. 1C). Both were significantly upregulated in knockdown cells, confirming that KAP1 is necessary not only for the establishment but also for the maintenance of HCMV latency in human HSC.

### HCMV latency in HSC correlates with KAP1-mediated SETDB1 recruitment and H3K9 trimethylation

To distinguish direct from indirect effects in the KAP1-mediated control of HCMV latency, the inventors performed KAP1-specific chromatin immunoprecipitation-deep sequencing (ChIP-seq) analyses on material isolated from human CD34+ HSC at 7 days post-TB40-E infection. Using a stringent peak-calling algorithm, the inventors identified 28 major KAP1-enriched regions in the HCMV genome. Supporting the functional significance of KAP1 recruitment to latent HCMV genomes, ChIP-PCR analyses also detected SETDB1 and H3K9me3 at these KAP1-enriched regions. Furthermore, in line with their former demonstration that heterochromatin formation can spread several tens of kilobases away from primary KAP1 docking sites (Groner et al., 2010), H3K9me3 was found at significant distances from these major KAP1 peaks, and particularly covered the major immediate early promoter (MIEP), the viral origin of lytic replication (OriLyt) and the UL112 gene. In contrast, the repressive mark was not found at genes known to be expressed during latency such as UL138 or LUNA (Reeves and Sinclair, 2013). Importantly, SETDB1 recruitment and H3K9 trimethylation were lost when KAP1 was depleted by RNA interference prior to HSC infection, supporting a model whereby the corepressor recruits the histone methyltransferase responsible for imposing the repressive histone mark (Fig. 2).

### A KAP1 phosphorylation switch governs HCMV progression from latency to lytic replication

TB40-E induces a full lytic cycle in MRC5 cells, where its genome accordingly fails to associate with SETDB1 and does not bear the H3K9me3 repressive mark (Fig. 3A). However, the inventors surprisingly found that, in these cells, KAP1 was still bound to the TB40-E episome (Fig. 3B). This indicated that HCMV replication can occur in spite of corepressor binding. To probe this issue further, the inventors infected CD34+ cord blood and MRC5 cells with the HCMV AD 169 laboratory strain, which is incapable of inducing latency (Goodrum et al., 2007 ; Saffert et al., 2010). At day 7 post-infection, while the productively transcribed AD169 genome carried as expected neither SETDB1 nor H3K9Me3, its still was bound by KAP1 in both cell types as robustly as the latent TB40-E strain in HSC (Fig. S3AB). Therefore, it is not KAP1 recognition but rather the secondary recruitment of its SETDB1 effector, and likely of other KAP1-associated heterochromatin inducers such as HP1, which is responsible for HCMV latency. Within the context of DNA damage, ATM phosphorylates KAP1 on serine 824, thereby reducing its ability to bind SETDB1 hence its repressor potential (Noon et al., 2010 ; White et al., 2012; White et al., 2006). The inventors thus investigated whether this modification could alter the consequences of KAP1 recruitment to the HCMV genome. Using immunofluorescence and co-staining with IE viral gene products-specific antibodies, the inventors detected S₈₂₄ phosphoKAP1 in 100% of TB40-E- or AD169-infected MRC-5 fibroblasts, but not in uninfected cells (Fig.3CD). However, the KU55933 ATM inhibitor did not prevent KAP1 phosphorylation, indicating that this kinase was not involved (Fig. S3D). The inventors then turned to the mammalian target of rapamycin (mTOR), because activation of this kinase was previously demonstrated to occur during HCMV replication (Clippinger and Alwine, 2012; Clippinger etal., 2011; Poglitsch et al., 2012). When HCMV-infected MRC-5 cells were treated with the mTORs inhibitors rapamycin or Torin1 (Thoreen et al., 2009; Thoreen and Sabatini, 2009), the phosphoKAP1-specific immunofluorescence signal was suppressed (Fig. 3E). Torin1-preventable S₈₂₄phosphoKAP1 accumulation was also documented in CD34+ HSC infected with the replicative AD169 strain (Fig. 3FG). Noteworthy, in this setting, the IE protein-specific signal was also reduced by the mTOR inhibitor, suggesting that IE viral gene expression was repressed. Finally, when CD34+ HSC were inoculated with the TB40-E strain, infected cells, identified by their loss of expression of the surface protein MRP1 as recently described (Weekes et al., 2013), failed to stain with the S₈₂₄phosphoKAP1-specific antibody (Fig. 3H). Importantly, global KAP1 levels were not modified by HCMV infection (Fig. S3GHI), and some KAP1 phosphorylated on serine 473 was detected in MRC5 cells, but this was independent of HCMV infection, as well as Torin1-resistant). These data suggested that the form of KAP1 bound to the actively transcribed HCMV genome might be a repression-inactive phosphorylated derivative. Confirming this hypothesis, the TB40-E genome could be immunoprecipitated with S₈₂₄phosphoKAP1-specific antibodies in productively infected MRC-5 fibroblasts but not in latently infected HSC, with a pattern of genomic recruitment identical to that delineated with the global KAP1 antibody in both cell types (Fig. 3IJ). Furthermore, S₈₂₄phosphoKAP1 was similarly found associated with the replicating AD169 strain in both MRC5 cells and CD34+HSC.

### HCMV can be forced out of latency by KAP1 genetic or pharmacological manipulation

The phosphomimetic S₈₂₄E-KAP1 derivative was previously demonstrated to lack repressor ability owing to a defect in SETDB1 recruitment (White et al., 2012). The inventors thus overexpressed shRNA-resistant forms of this mutant protein or of its wild-type counterpart in CD34+ HSC depleted for endogenous KAP1 by RNA interference, and infected the resulting cells with the latency-competent TB40-E HCMV strain (Fig. 4A). Efficient levels of complementation were obtained with both alleles as assessed by RT-PCR of *Kap1*-specific RNA (Fig. S4A). Furthermore, measurements of the immediate early UL122, UL123, early UL54 and late UL94 viral transcripts confirmed that the shRNA-resistant wild-type protein could successfully revert the effect of the knockdown by restoring HCMV latency. In contrast, S₈₂₄E-KAP1 overexpression did not repress viral transcription, and correspondingly did not reinstate H3K9me3 on the viral DNA (Fig. 4AB). The inventors then tested its impact on TB40-E latency in control HSC, that is, in the presence of wild-type endogenous KAP1. In this setting, the phosphomimetic mutant also stimulated viral gene expression, albeit to a smaller degree (Fig. 4C). This strongly suggested that this mutant was not only defective for the maintenance of HCMV latency, but that it was capable of a dominant negative effect over the wild-type protein, consistent with a model whereby KAP1 phosphorylation prevents viral gene repression.

To explore further this issue, the inventors used a pharmacological approach. Because previously published (White et al., 2012) and their own (Fig. 3) data indicated that either ATM or mTOR could induce KAP1 S₈₂₄ phosphorylation, they used known activators of each one of these two kinases. When HSC infected 5 days earlier with TB40-E were incubated with either the ATM activator chloroquine (Bakkenist and Kastan, 2003) or the mTOR activator MHY-1485 (Choi et al., 2012), S₈₂₄phosphoKAP1-positive cells were detected by immunofluorescence, which further revealed that these cells were positive for IE antigens (Fig. 5A). It suggested that the virus harbored by these cells had exited latency but also indicated, based on the detection of phosphorylated S6K, a marker of mTOR activation (Clippinger et al., 2011), in all MHY-455- treated cells, that KAP1 became phosphorylated through the combined action of either kinase and some HCMV-encoded factor. Confirming TB40-E de-repression, levels of viral transcripts also increased in drug-treated cells, only transiently after a single dose of drug but steadily if three doses were administered over 5 days (Fig. 5B). Furthermore, single doses of the ATM and mTOR activators prevented the progressive drop in viral DNA that was observed in control cells, and repeated doses triggered an augmentation in viral DNA copies, indicative of genome replication, importantly without triggering HSC differentiation (Fig.5C). Finally, when the supernatant of TB40-E-infected HSC exposed to either chloroquine or MHY-1485 was used to inoculate MRC5 fibroblasts, it induced the accumulation of viral DNA and IE antigens in these targets, demonstrating that the production of replication-competent virus had been induced by the ATM- or mTOR-activating treatment of the latently infected HSC (Fig. 5D).

### Conclusion

The inventors have figured out mechanisms of HCMV latency by revealing that, in latently infected hematopoietic stem cells, the master co-repressor KAP1 recruits SETDB1 to the viral genome, where this histone methyltransferase deposes the H3K9me3 repressive mark. These findings correlate the observation that, in latently infected CD34+ HSC or circulating monocytes isolated from seropositive individuals, chromatin at the HCMV MIEP bears H3K9me3 and HP1, another KAP1-interacting protein (Sinclair, 2010).

Further, these findings indicate that KAP1 is necessary for both the establishment and the maintenance of HCMV.

More precisely, the inventors have shown that the HCMV exits latency when KAP1 becomes phosphorylated on serine 824, which blocks its ability to recruit SETDB1 and hence abrogates its co-repressor potential.

Finally, the inventors identified mTOR as responsible for this KAP1 phosphorylation switch.

Overall, the inventors have shown that HCMV latency can be externally controlled by agents acting on this enzyme or on ATM, another KAP1 kinase.

More precisely, HCMV can be forced out of transcriptional dormancy by inducing the activation of either mTOR or ATM, another kinase targeting KAP1 on serine 824, in latently infected HSC.

Finally, the inventors have developed new avenues to eradicate HCMV by combining a compound such as mTOR or ATM activation with immune- or drug-based approaches aimed at killing HCMV-infected cells. While the systemic administration of current mTOR or ATM activators may cause important side effects, the ex vivo treatment of HCMV-positive transplants, for instance bone marrow or HSC purified therefrom, represent a promising strategy for purging latently infected cells prior to engraftment.

## Claims

1. Use of a composition for purging *ex vivo* isolated organs or isolated cells from latent human cytomegalovirus (HCMV), wherein said composition comprises:
- an agent selected in the group consisting of a KAP1 protein with a phosphorylated serine on position 824, a kinase which phosphorylates KAP1 on serine 824, an activator of said kinase, and a phosphatase inhibitor targeting KAP1 on serine position 824; and
- an anti-CMV agent.

2. Use according to claim 1, wherein said agent is a kinase selected in the group consisting of mTOR and ATM.

3. Use according to claim 1 or 2, wherein said activator of the kinase is selected from activators of mTOR and activators of ATM.

4. Use according to claim 3, wherein said activator is selected from MHY-1485 and chloroquine.

5. Use according to any one of claims 1 to 4, wherein said anti-CMV agent is selected from monoclonal antibodies, antiviral drugs and cytotoxic agents.

6. Use according to any one of claims 1 to 5, wherein said cytotoxic agent is vincristine.

7. Use according to any one of claims 1 to 6, said isolated organ is a graft organ.

8. Use according to any one of claims 1 to 7, wherein said isolated cells are blood cells, preferably hematopoietic stem cells.

9. Use according to any one of claims 1 to 7, wherein said isolated organ is selected from the bone marrow, kidney, liver, pancreas, heart, and lung.

10. A compound for use in a method for treating HCMV infection, wherein said compound prevents KAP1 phosphorylation on serine 824.

11. A compound for use according to claim 9, wherein said compound is selected in the group consisting of:
- inhibitors of a kinase which phosphorylates KAP1 on serine 824, and
- inhibitors of activators of kinases which phosphorylate KAP1 on serine 824,
- phosphatases targeting KAP1 on position 824, and
- activators of phosphatases targeting KAP1 on position 824.

12. A compound for use according to claim 10 or 11, wherein said compound is an inhibitor of a kinase which phosphorylates KAP1 on serine 824, said kinase being selected from mTOR and ATM.

13. A compound for use according to claim 10 or 11, wherein said phosphatase targeting KAP1 on position 824 is selected from PP1B and PP4C.

14. A compound for use according to claim 10 or 11, where said activator of phosphatases targeting KAP1 on position 824 is ceramide.
